# EUROPÄISCHE PATENTANMELDUNG

(11) **EP 0 931 557 A1**
(43) Veröffentlichungstag der Anmeldung: **28.07.1999**
(21) Anmeldenummer: 99101177.6
(22) Anmeldetag: 22.01.1999
(51) Int. Cl.: A61M 16/08, A61M 16/00

(54) **Atembeutel für die Beatmung eines Patienten**

(30) Priorität: 27.01.1998 DE 29801254 U
(71) Anmelder: Scheu, Rolf Rainer, 60488 Frankfurt/Main (DE)
(72) Erfinder: Scheu, Rolf Rainer, 60488 Frankfurt/Main (DE)
(74) Vertreter: Müller-Gerbes, Margot, Dipl.-Ing.

(57) **Zusammenfassung**

Die Erfindung betrifft einen Atembeutel (1) aus einem weichelastischen Material für die Beatmung eines Patienten mit an zwei gegenüberliegenden Enden des Atembeutels (1) ausgebildeten Anschlußstutzen (2,3) zum Einsetzen des Atembeutels (1) als Zwischenglied in eine Atemgasleitung (6a,6b), wobei jeder Anschlußstutzen (2,3) an dem jeweiligen dem Atembeutel (1) abgewandten Ende einen Anschlußkonus (51,52) zum Auf- oder Einstecken der anzuschließenden Atemgasleitung (6a,6b) aufweist.

## Beschreibung

Die Erfindung betrifft einen Atembeutel aus einem weichelastischen Material für die Beatmung eines Patienten.

Derartige Atembeutel sind vielfältig bekannt und werden bei der Beatmung eines Patienten in der entsprechenden Atemluftleitung vorgesehen, um den von einer Atemgasquelle unter Druck herangeführten Luftstrom in seinem Druck zu reduzieren und während des Einatmens bzw. Ausatmens des Patienten einen Puffer für das Atemgas zu bilden. Anhand der Füllung und Entleerung des Atembeutels mit Atemgas während des Ein- und Ausatmens des Patienten ist der zur Atemgasversorgung und Beatmung erforderliche Volumenstrom an Atemgas erkennbar und kann entsprechend an der Atemgasquelle eingestellt werden.

Aufgabe der Erfindung ist es, Atembeutel bezüglich ihrer Funktionalität zu verbessern. Der Atembeutel soll darüber hinaus auf einfache Weise herstellbar sein und eine verbesserte Kontrolle des erforderlichen einzustellenden Volumenstroms an Atemgas für den Patienten ermöglichen.

Diese Aufgabe wird erfindungsgemäß durch Ausgestaltung eines

Atembeutels mit den Merkmalen des Patentanspruches 1 gelöst. Vorteilhafte Weiterbildungen der Erfindung sind in den Unteransprüchen angegeben.

Der erfindungsgemäß vorgeschlagene Atembeutel für die Beatmung eines Patienten ist aus einem weichelastischen Material hergestellt und weist an zwei gegenüberliegenden Enden des Atembeutels Anschlußstutzen zum Einsetzen des Atembeutels als Zwischenglied in eine Atemgasleitung auf, so daß in den Atembeutel zwei Anschlußstutzen an gegenüberliegenden Seiten einmünden, die als Ein- und Ausgang für das dem Patienten mittels der Atemgasleitung zuzuführende Atemgas dienen. Zur Erzielung einer universellen Anschließbarkeit wird vorgeschlagen, daß jeder Anschlußstutzen an dem dem Atembeutel abgewandten Ende einen Anschlußkonus zum Auf- oder Einstecken der jeweiligen Atemgasleitung aufweisen. Derartige Anschlußkonen sind in der Beatmungstechnik weit verbreitet und standardisiert, so daß sich die meisten üblicherweise verwendeten Atemgasleitungen, Schläuche, Winkelstücke, Masken usw. an den erfindungsgemäßen Atembeutel und dessen Anschlußstutzen durch einfache Steckverbindungen anschließen lassen.

Der erfindungsgemäße Atembeutel ermöglicht die Ausbildung einer Atemgasleitung mit einem Reservoir für das Atemgas, das dem Patienten zugeführt werden soll, in dem der Atembeutel als Zwischenglied in einer Inline-Anordnung in der Atemgasleitung angeordnet ist und Teil der Atemgasleitung bildet. Die Strömungsrichtung des Atemgasvolumenstromes wird hierbei von einer Atemgasquelle über die Atemgasleitung kommend und durch den Atembeutel hindurchströmend und weiter über die Atemgasleitung bis zum Patienten nicht verändert.

Hierbei kann beispielsweise vorgesehen sein, daß die verwendeten Anschlußkonen des Atembeutels als Innen- oder Außenkonus nach DIN/ISO 5356/1 ausgebildet sind, so daß sie mit sämtlichen nach dieser weit verbreiteten Norm ausgebildeten konischen Anschlußmitteln von Atemgasleitungen etc. universell verbindbar sind.

Durch die Ausbildung des Atembeutels mit an zwei gegenüberliegenden Enden desselben ausgebildeten Anschlußstutzen wird dieser bei Einsatz innerhalb der Atemgasleitung zur Beatmung des Patienten zu einem Inline-Reservoirbeutel, in dessen Inneren ständig ein Reservoir an Atemgas für die Beatmung des Patienten vorgehalten wird und während des Atemvorganges lediglich eine Teilmenge aus dem Atembeutel entnommen wird. Hierbei wird sowohl der Druck in der Atemgasleitung, unter welchem das Atemgas von der Atemgasquelle zum Patienten herangeführt wird, abgebaut, in dem sich der Atembeutel auffüllt, wie auch die Entnahme einer benötigten Menge an Atemgas beim Einatmen des Patienten ständig ermöglicht ist. Die Einstellung des erforderlichen Volumenstroms an Atemgas wird hierbei beispielsweise gegenüber bekannten Atembeuteln mit lediglich einem als Einlaß und Auslaß dienenden Anschlußstutzen schneller und direkter angezeigt.

Zur Anpassung an unterschiedliche Lungenvolumina von Patienten kann darüber hinaus der erfindungsgemäße Atembeutel mit unterschiedlichen Volumina ausgebildet werden, beispielsweise angepaßt an den Einsatz bei Kleinkindern, Kindern, Jugendlichen und Erwachsenen, um eine schnellere und genauere Anzeige des erforderlichen Volumenstroms an Atemgas zu ermöglichen.

Der erfindungsgemäße Atemgasbeutel bildet für den Atemgasstrom zum zu beatmenden Patienten ein Durchflußreservoir, welches mit Atemgas angefüllt ist.

In einer Ausführungsform der Erfindung werden die an den beiden gegenüberliegenden Enden des Atembeutels ausgebildeten Anschlußstutzen jeweils von einem in den Atembeutel hineinragenden und am Atembeutel haftfest und dicht befestigten Rohrabschnitt gebildet, an dessen dem Atembeutel abgewandten Ende der Anschlußkonus angeformt ist. Dieser Anschlußstutzen ist einstückig und kann als Spritzgußteil aus dem gleichen Kunststoff wie der Atembeutel hergestellt sein und beispielsweise mit den Atembeutel mittels einer Schweißverbindung verbunden sein.

Eine weitere Ausführungsform der Erfindung sieht vor, daß die Anschlußstutzen jeweils von einem in den Atembeutel hineinragenden Rohrabschnitt gebildet sind und je ein Einsteckstück mit Anschlußkonus vorgesehen ist, welches in das jeweilige, dem Atembeutel abgewandte Ende des Rohrabsabschnittes einsteckbar ist.

In diesen beiden möglichen Ausführungsformen der Erfindung wird der den Anschlußstutzen bildende Rohrabschnitt mit dem eigentlichen Beutel bei dessen Herstellung verschweißt, was eine besonders einfache Herstellung ermöglicht. Der Anschluß der Atemgasleitungen an die Anschlußstutzen erfolgt sodann entweder über den integral an dem Rohrabschnitt angeformten Anschlußkonus oder über zusätzliche Einsteckstücke mit Anschlußkonus, die in den Rohrabschnitt eingesteckt werden und an ihrem freien Ende den Anschlußkonus für die Atemgasleitung bilden.

Die Länge des Rohrabschnittes kann unterschiedlich sein, bei langen Rolhrabschnitten kann auch ein schlauchförmiger Rohrabschnitt, d. h. ein elastischerer und biegsamer Rohrabschnitt anstelle eines steiferen vorgesehen werden.

In einer weiteren Ausführungsform der Erfindung ist vorgesehen, daß die Anschlußstutzen mit Anschlußkonus integral an den Atembeutel angeformt sind und integral mit diesem bei der Herstellung desselben erzeugt werden. Auch hierbei ist eine besonders einfache Herstellung ermöglicht, die den universellen Anschluß von Atemgasleitungen an die Anschlußkonen der Anschlußstutzen ermöglicht.

Selbstverständlich ist es möglich, erfindungsgemäße Atembeutel mit zwei gleich ausgebildeten Anschlußstutzen oder auch unterschiedlichen Anschlußstutzen herzustellen.

Vorteilhaft weist der Atembeutel einen Anschlußstutzen mit als Innenkonus ausgebildetem Anschlußkonus und einen Anschlußstutzen mit als Außenkonus ausgebildetem Anschlußkonus auf. Durch diese Ausgestaltung läßt sich der erfindungsgemäße Atembeutel an jeder beliebigen Verbindungsstelle einer Atemgasleitung, die durch Einstecken entsprechend ausgebildeter konischer Anschlußmittel verbunden wird, in den Atemgasweg für die Beatmung des Patienten einfügen.

Der erfindungsgemäße Atembeutel kann beispielsweise aus einem weichelastischen Kunststoff, wie Weich-PVC hergestellt sein.

Auch ist es möglich, daß er aus einem mehrfach sterilisierbaren, weichelastischen Material, wie Polyurethan oder Silikon, mit einer Temperaturbeständigkeit von mindestens 95 °C hergestellt ist. In diesem Fall ist durch die erhöhte Temperaturbeständigkeit von mindestens 95 °C die Möglichkeit gegeben, den erfindungsgemäßen Atembeutel beispielsweise mittels Dampfsterilisation mehrfach erneut zu sterilisieren und daher mehrfach zu verwenden.

Eine weitere erfindungsgemäße Ausgestaltung des Atembeutels zeichnet sich dadurch aus, daß er an einem Anschlußstutzen mit einer Atemgasleitung, die an ihrem freien Ende mit einer Atemgasquelle verbindbar ist und an dem anderen Anschlußstutzen mit einer zu einem Patienten führbaren Atemgasleitung, an deren freien Ende eine Nasenmaske anschließbar ist, ausgestattet ist.

Die Erfindung wird nachfolgend anhand von Ausführungsbeispielen in der Zeichnung näher erläutert. Es zeigen
- Figur 1: die schematische Schnittaufsicht auf einen erfindungsgemäßen Atembeutel,
- Figur 2: die schematische Schnittaufsicht auf eine weitere Ausführungsform des erfindungsgemäßen Atembeutels,
- Figur 3: die schematische Schnittaufsicht auf eine weitere Ausführungsform des erfindungsgemäßen Atembeutels,
- Figur 4 u. 5: Ansicht zweier weiterer Ausführungsform des erfindungsgemäßen Atembeutels,
- Figur 6: eine Atemgasleitung mit Inline-Atembeutel und
- Figur 7: eine Atemgasgleitung mit Atembeutel und Nasenmaske.

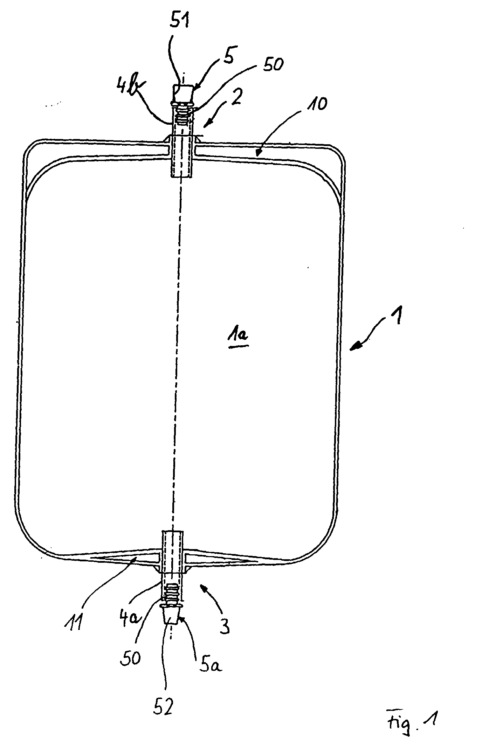

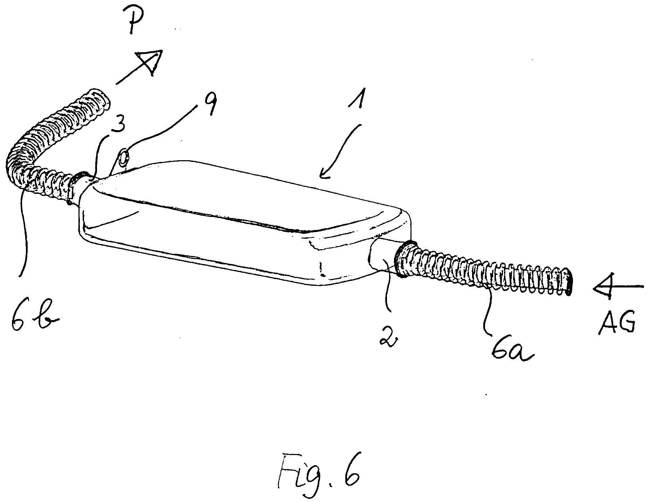

In der Figur 1 ist ein Atembeutel 1 dargestellt, der als Reservoirbeutel zur Druckminderung und zum Bereitstellen eines Reservoirs an Atemgas für die Beatmung eines Patienten verwendet werden kann. Der Atembeutel 1 besteht aus einem befüllbaren Beutelkörper 1a zur Aufnahme und Abgabe des Atemgas sowie zwei an jeweils gegenüberliegenden Enden des Atembeutels 1 ausgebildeten und in den Beutelkörper 1a einmündenden Anschlüsse 2, 3 für den Anschluß von Atemgasleitungen 6a, 6b, die von einer Atemgasquelle AG zum zu beatmenden Patienten P führen, siehe Fig. 6. Die Flußrichtung des Atemgases ist in Pfeilrichtung von der Atemgasquelle AG kommend und über den ersten Anschlußstutzen 2 in den Atembeutel 1 und den Atembeutel 1 durchströmend, wobei der Atembeutel sich anfüllt, bei dem zweiten Anschlußstutzen 3 aus dem Atembeutel 1 heraus und über die anschließende Atemgasleitung 6b zum Patienten P hin. Bei Beaufschlagung des Atembeutels mit Atemgas unter Druck füllt sich der Atembeutel 1 und mindert somit für den Patienten den Druck des Atemgases, wobei der Atembeutel 1 gleichzeitig als Atemgasreservoir für den Patienten dient, und eine Teilmenge des Atemgases aus dem Beutelkörper 1a über einen der Anschlußstutzen zur Beatmung des Patienten entnommen wird.

Im dargestellten Ausführungsbeispiel gemäß Figur 1 ist der Atembeutel aus Abschnitten einer Kunststoffolie, beispielsweise aus Weich-PVC oder Polyurethan hergestellt und mittels umlaufender Schweißnähte 10, 11 unter Ausbildung des Beutelkörpers 1a zu dem Atembeutel 1 verschweißt. Die an den gegenüberliegenden Enden des Atembeutels 1 jeweils ausgebildeten Ein- und Ausgänge für den Anschluß der Atemgasleitungen werden von in den Atembeutel 1 eingesetzten und bei der Verschweißung der Folienabschnitte mit eingeschweißten Rohrabschnitten 4a, 4b gebildet, was eine besonders einfache Herstellung ermöglicht. Der erfindungsgemäße Atembeutel weist somit einen Durchgangsweg mit separatem Eingang und separatem Ausgang für das Atemgas auf.

Zum Anschluß der Atemgasleitungen, die standardisiert mit einem Anschlußkonus nach DIN/ISO 5356/1 ausgebildet sind, werden als Konnektor ausgebildete Einsteckteile 5, 5a in das jeweilige freie Ende der Rohrstutzen 4a, 4b eingeschoben. Zu diesem Zweck weisen die Einsteckteile 5, 5a jeweils einen Einsteckbereich 50, 50a auf, der in den Rohrstutzen 4a bzw. 4b eingeschoben wird und dort eine Fixierung bewirkt. An ihrem anderen jeweils dem Atembeutel 1 abgewandten freien Ende sind die Einsteckteile 5, 5a mit einem Anschlußkonus 51, 52 versehen, die nach DIN/ISO 5356/1 ausgebildet sind und dem bei der Beatmung eines Patienten üblichen Standard entsprechen, so daß die handelsüblichen Atemgasleitungen und weitere Geräte, wie Atemgasquellen, wie Winkelstücke und dergleichen mehr mit ihren Anschlußmitteln, die ebenfalls der DIN/ISO 5356/1 entsprechen an den Atembeutel 1 anschlielßbar sind. Im gezeigten Ausführungsbeispiel ist der Anschlußkonus 51 des Einsteckstückes 5 als Innenkonus zum Einstecken einer Atemgasleitung nach DIN/ISO 5356/1 ausgebildet, während der Anschlußkonus 52 des Einsteckteiles 5a als Außenkonus ausgebildet ist und das Aufstecken einer entsprechend ausgebildeten Atemgasleitung mit Anschlußkonus ermöglicht. Auf diese Weise kann der in der Figur 1 dargestellte Atembeutel an jeder beliebigen mit Anschlußkonen gemäß DIN/ISO 5356/1 geschlossenen Verbindungsstelle innerhalb des Beatmungssystems zwischen der Atemgasquelle und dem Patienten auf einfache Weise in die Atemgasleitung 6a, 6b, siehe Figur 6 eingefügt werden.

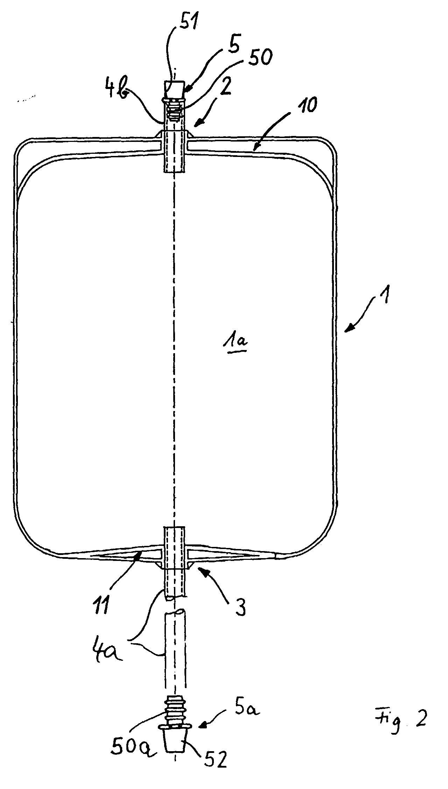

In Abwandlung dieses in der Figur 1 dargestellten Ausführungsbeispieles ist in der Figur 2 ein Atemgasbeutel 1 dargestellt, der im wesentlichen gleich aufgebaut ist, bei dem jedoch der Anschlußstutzen 3 von einem längeren Schlauchabschnitt als Rohrstutzen 4a gebildet ist, der einseitig in den Atembeutel 1 eingesetzt und verschweißt ist und an seinem anderen, dem Atembeutel 1 abgewandten Ende ein Einsteckteil 5a mit Anschlußkonus 52 für den Anschluß einer Atemgasleitung aufweist. Der verlängerte Rohrstutzen 4a dient hierbei als Verlängerung der Atemgasleitung, was in bestimmten Anwendungsfällen von Vorteil ist. Es ist auch möglich, an beiden Ein- und Ausgängen des Atembeutels verlängerte Rohrstutzen 4a, 4b vorzusehen.

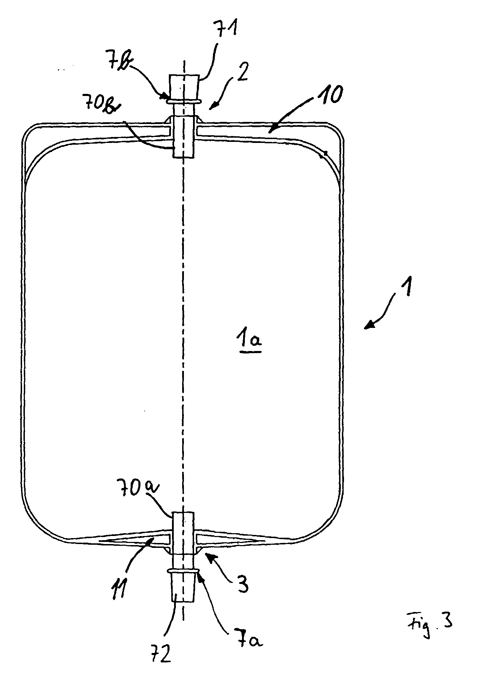

Eine weitere Ausführungsform des Atembeutels 1 ist in der Figur 3 dargestellt. Hierbei sind die Anschlußstutzen 2, 3 von einem einstückig ausgebildeten Konnektor 7a bzw. 7b gebildet, die einen in den Beutelkörper 1a des Atembeutels 1 einmündenden Rohrabschnitt 70a bzw. 70b umfassen, der mit dem Atembeutel 1 verschweißt ist, und einen Anschlußkonus 72, 71 für den Anschluß einer entsprechend ausgebildeten Atemgasleitung mit Anschlußkonus, beispielsweise nach DIN/ISO 5356/1 integral angeformt, aufweisen. Im dargestellten Ausführungsbeispiel ist der Anschlußkonus 71 am Anschlußstutzen 2 als Innenkonus ausgebildet, während der Anschlußkonus 72 am Anschlußstutzen 3 aus Außenkonus ausgebildet ist und somit die bereits beschriebene einfache Einfügung des Atembeutels 1 in die den Atemgasweg zum Patienten bildenden Atemgasleitung ermöglicht.

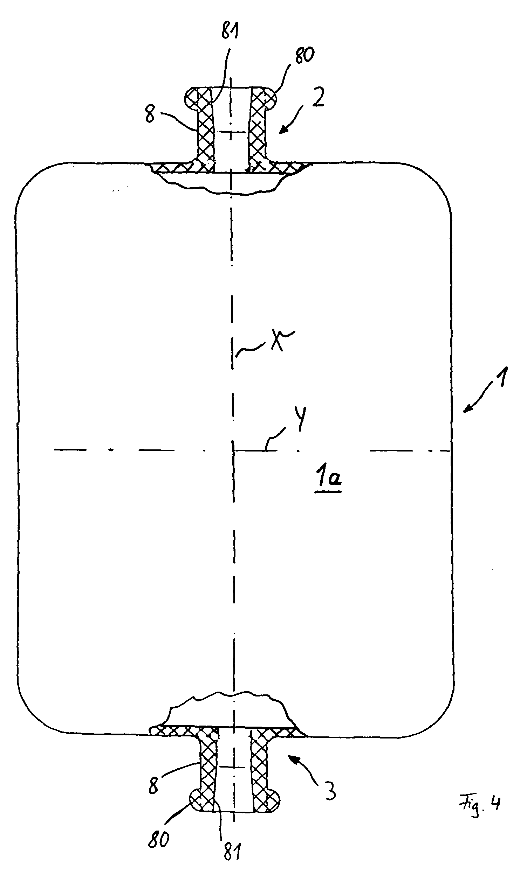

Eine weitere Ausführungsform des Atembeutels 1 ist in der Figur 4 dargestellt. Dieser Atembeutel 1 kann beispielsweise vollflächig aus einem weichelastischen Material, wie Silikon mit einer Temperaturbeständigkeit von mindestens 95 °C hergestellt sein, wodurch eine mehrfache Wiederverwendung mittels Dampfsterilisation ermöglicht ist. Bei diesem Atembeutel 1 sind die Anschlußstutzen 2, 3 integral an den Beutelkörper 1a angeformt und weisen an ihrem jeweiligen freien Ende einen über einen zylindrischen Abschnitt 8a, b vorstehenden umlaufenden Wulst 80a, b auf. Bei diesem Ausführungsbeispiel sind beide Anschlußstutzen 2, 3 mit einem Anschlußkonus 81 als Innenkonus beispielsweise nach DIN/ISO 5356/1 ausgebildet, in die entsprechende ausgebildete Atemgasleitungen einsteckbar sind. Der Atembeutel gemäß Figur 4 ist symmetrisch in bezug auf seine Mittelachse X und auch in bezug auf seine Querachse Y ausgebildet, auch andere unsymmetrische Formen des Atembeutels sind möglich.

Eine mehrfache Sterilisation und Wiederverwendung des Atembeutels kann auch durch Herstellung desselben aus einem entsprechend temperaturbeständigen Polyurethan erzielt werden.

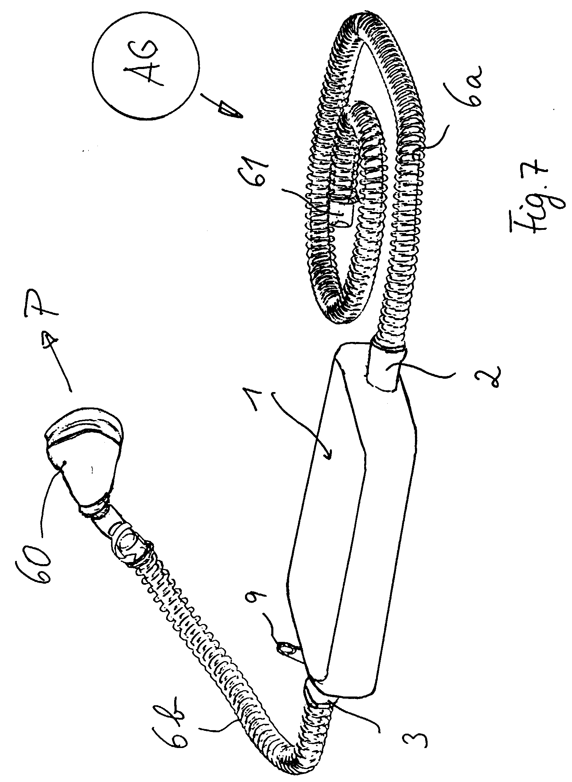

Durch die Ausbildung des Atembeutels mit an zwei gegenüberliegenden Enden des Beutelkörpers 1a ausgebildeten Anschlußstutzen 2, 3 dient der Atembeutel 1 als Inline-Reservoirbeutel für das Atemgas des zu beatmenden Patienten. In der Figur 6 und 7 ist die völlig neue und für den Patienten vorteilhafte Ausbildung einer Atemgasleitung, 6a, 6b, die von einer nicht dargestellten Atemgasquelle AG zum Patienten P führt, dargestellt. Gemäß der Erfindung wird ein Inline-Reservoir innerhalb der Atemgasleitung durch Zwischenschaltung des Atembeutels 1 gebildet, ohne daß der Strömungsweg des Atemgases durch die Atemgasleitung unterbrochen oder die Strömungsrichtung geändert wird. Der Atembeutel 1 ist hier mit zwei an einander gegenüberliegenden Seiten des Beutels angeordneten und ausgebildeten Öffnungen versehen, die als Anschlußstutzen 2, 3 ausgebildet sind und als Eingang bzw. Ausgang dienen und an die die Atemgasleitungen 6a bzw. 6b angeschlossen sind. Der Atembeutel bildet erfindungsgemäß ein Teilstück der Atemgasleitung. Der zum Patienten führende Teil der Atemgasleitung 6b ist am Ende mit einer Nasenmaske 60 ausgerüstet. Am anderen Ende 61 der Atemgasleitung ist die Atemgasquelle AG anschließbar` Der Atembeutel 1 kann des weiteren im Bereich eines Anschlußstutzens eine aufgeschobene Lasche 9 mit Aufhängeöse aufweisen. Die in der Figur 7 aus Atembeutel 1 mit zwei Anschlußstutzen 2, 3 mit angeschlossenen Atemgasleitungen 6a und 6b einschließlich Nasenmaske 60 dargestellte Anordnung stellt in ihrer Gänze die erfindungsgemäße Vorrichtung zur Verbesserung der Beatmung eines Patienten mit verbesserter Kontrolle und damit Einstellbarkeit des erforderlichen Volumenstromes an Atemgas für den Patienten dar. Der Atembeutel ist in dem entsprechend seinem vorkonfektionierten Volumen entsprechenden Umfange mit Atemgas anfüllbar. Ein Dehnen des Atembeutels ist nicht vorgesehen. Die Herstellung aus einem weichelastischen Material, wie Weich-PVC, Silikon, Polyurethan dient dem angenehmeren und leichteren Handling.

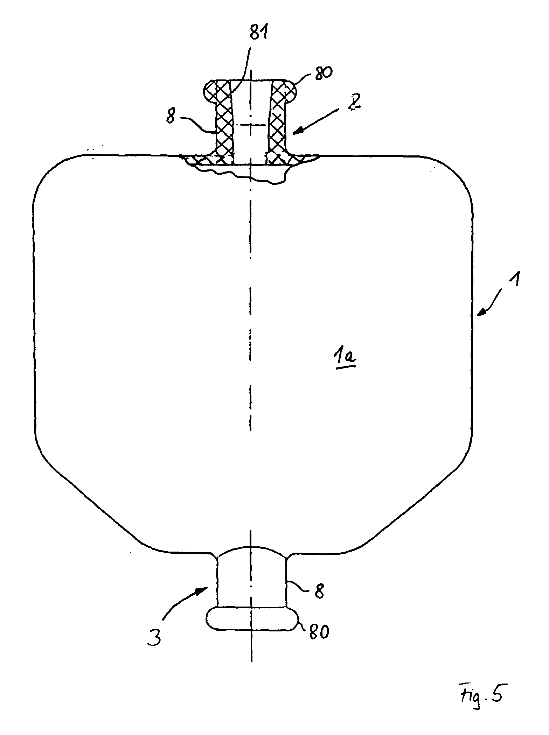

Um hierbei eine Anpassung an unterschiedlichem Lungenvolumina verschiedener Patienten in Abhängigkeit z. B. von ihrer Körpergröße zu erzielen, kann der Atembeutel mit Beutelkörpern 1a unterschiedlicher Volumina ausgebildet sein. Das Volumen des Beutelkörpers 1a, welches dem Patienten als Atemgasreservoir dient, ist demgemäß einfach an dessen spezifischen Atemgasbedarf angepaßt. Dementsprechend zeigt die Figur 4 einen Atembeutel 1, wie er beispielsweise für die Beatmung eines erwachsenen Patienten verwendet werden kann. Demgegenüber zeigt die Figur 5 einen Atembeutel 1 mit gegenüber dem Atembeutel 1 gemäß Figur 4 verringerten Volumen des Beutelkörpers 1a, so daß dieser in der Figur 5 dargestellte Atembeutel beispielsweise für die Beatmung eines Kindes mit entsprechend geringerem Lungenvolumen und damit Atemgasbedarf einsetzbar ist.

Die vorangehend dargestellten Ausführungsformen des erfindungsgemäßen Atembeutels stellen lediglich beispielhafte Ausgestaltungen dar. Die Form der Atembeutel kann flächig, z. B. als Folienbeutel, wie in Figur 1 oder auch dreidimensional, wie in Figur 4, eckig, rund oder oval sein, auch ist es möglich, den Beutel zu falten.

Der erfindungsgemäße Atembeutel ermöglicht eine Inline-Anordnung als ein Reservoir bildendes Zwischenglied in einer Atemgasleitung und ermöglicht dazu eine verbesserte kontrollierbare Beatmung eines Patienten. Ein universeller Einsatz, ist durch die Ausrüstung mit gängigen Anschlußmitteln mit Anschlußkonus, wie bei Atemgasleitungen in standardisierter Form verbreitet, möglich.

## Patentansprüche

1. Atembeutel aus einem weichelastischen Material für die Beatmung eines Patienten mit an zwei gegenüberliegenden Enden des Atembeutels ausgebildeten Anschlußstutzen zum Einsetzen des Atembeutels als Zwischenglied in eine Atemgasleitung, wobei jeder Anschlußstutzen an dem jeweiligen dem Atembeutel abgewandten Ende einen Anschlußkonus zum Auf- oder Einstecken der anzuschließenden Atemgasleitung aufweist.

2. Atembeutel nach Anspruch 1, **dadurch gekennzeichnet,** daß der Anschlußstutzen einstückig von einem in den Atembeutel hineinragenden und mit dem Atembeutel dicht verbundenen Rohrabschnitt mit einem angeformten über den Atembeutel nach außen vorstehenden Anschlußkonus gebildet ist.

3. Atembeutel nach Anspruch 1, **dadurch gekennzeichnet,** daß der Anschlußstutzen von einem in den Atembeutel hineinragenden und mit dem Atembeutel dicht verbundenen Rohrabschnitt gebildet ist und an dem über den Atembeutel nach außen vorstehenden Ende des Rohrabschnittes ein als Konnektor ausgebildetes Einsteckstück mit Anschlußkonus einsteckbar ist.

4. Atembeutel nach Anspruch 1, **dadurch gekennzeichnet,** daß der Anschlußstutzen mit Anschlußkonus integral an den Atembeutel angeformt ist.

5. Atembeutel nach einem der Ansprüche 1 bis 4**, dadurch gekennzeichnet,** daß der Anschlußkonus als Innen- oder Außenkonus nach DIN/ISO 5356/1 ausgebildet ist.

6. Atembeutel nach einem der Ansprüche 1 bis 5, **dadurch gekennzeichnet,** daß einer der beiden Anschlußstutzen mit als Innenkonus ausgebildetem Anschlußkonus und der andere Anschlußstutzen mit einem als Außenkonus ausgebildeten Anschlußstutzen ausgestattet ist.

7. Atembeutel nach einem der Ansprüche 1 bis 6, **dadurch gekennzeichnet,** daß er aus einem weichelastischen Kunststoff, wie Weich-PVC hergestellt ist und die Anschlußstutzen aus dem gleichen oder hiermit kompatiblen Kunststoff hergestellt sind, so daß eine homogene haftfeste und dichte Verbindung zwischen Atembeutel und Anschlußstutzen herzustellen ist.

8. Atembeutel nach einem der Ansprüche 1 bis 6, **dadurch gekennzeichnet,** daß er aus einem mehrfach sterilisierbaren weichelastischen Material, wie Polyurethan oder Silikon, mit einer Temperaturbeständigkeit von mindestens 95 °C hergestellt ist.

9. Atembeutel nach einem der Ansprüche 1 bis 8, **dadurch gekennzeichnet,** daß er ein dem Lungenvolumen des zu beatmenden Patienten entsprechendes befüllbares Innenvolumen aufweist.

10. Atembeutel nach einem der Ansprüche 1 bis 9, **dadurch gekennzeichnet,** daß er an einem Anschlußstutzen mit einer Atemgasleitung, die an ihrem freien Ende mit einer Atemgasquelle verbindbar ist und an dem anderen Anschlußstutzen mit einer zu einem Patienten führbaren Atemgasleitung, an deren freien Ende eine Nasenmaske anschließbar ist, ausgestattet ist.
